# EUROPEAN PATENT APPLICATION

(11) **EP 4 650 755 A1**
(43) Date of publication of application: **19.11.2025**
(21) Application number: 25167896.7
(22) Date of filing: 02.04.2025
(51) Int. Cl.: G01N 21/65, G01N 21/85, G01N 35/00

(54) **MULTIPLEXING OF A RAMAN ANALYZER BY USE OF A CARTESIAN ROBOT**

(30) Priority: 15.05.2024 US 202418664742
(71) Applicant: Endress+Hauser Optical Analysis, Inc., Ann Arbor, MI 48103 (US)
(72) Inventor: Strachan, David J., Timonium, 21093 (US); Moore, Thomas, 07751 Jena-Drackendorf (DE); WInter, Marc, 63571 Gelnhausen (DE)
(74) Representative: Endress + Hauser Group Services (Deutschland) AG+Co. KG

(57) **Abstract**

A method of performing an optical analysis on multiple vessels using a single optical analysis device includes placing on and in each vessel a protective sheath that will accept a probe head of the optical analysis device. The probe head may be easily inserted into and removed from the protective sheath. A Cartesian robot may move the probe head from vessel to vessel to perform the analyses. The system for the optical analysis include at least two vessels, an optical analyzer, and a Cartesian robot.

## Description

The present disclosure relates generally to a method of performing a Raman analysis of multiple containers using a single Raman probe.

In the current fields of biological and biochemical sciences, a bioreactor may be used to run some biologic or biochemical process. The biologic or biochemical process may be for the production of cells or organisms, or may be for the production of substances (chemicals, enzymes, etc.) by the organisms within the bioreactor.

As a part of the biologic or biochemical process, a Raman probe may be used to determine, with specificity, the contents of the bioreactor. In some applications, a Raman probe may be directly integrated into the bioreactor. But in other applications there may be a need for an array, a plurality, of smaller, e.g. single use, bioreactors, and the integration of a Raman probe into each of the array of bioreactors quickly becomes expensive. In these applications, the Raman analysis may therefore include extracting a sample (either manually or in an automated manner) from the bioreactor and passing that sample into a cuvette or flow cell of the Raman probe. The Raman analysis is then performed on the sample in the cuvette or flow cell.

Because the extraction tools and the cuvette or flow cell are the same for each Raman analysis, these items must be rinsed after each analysis. The rinsing process is time-consuming and off-line, and if sufficient rinsing is not performed, the process can introduce errors. And there are additional sources for errors, for example, mis-labeling, sample or bioreactor contamination, and a change of the sample while waiting for the measurements may all be possible errors in the Raman analysis process.

Therefore, there remains a need to simplify the process of performing Raman analyses on the contents of individual bioreactors in an array of bioreactors.

According to at least one embodiment of the present disclosure, a system for optical analysis comprises at least two vessels, each vessel embodied to contain a liquid or gaseous process and each vessel having a port enabling access to an interior of the respective vessel; at least two barbs, each barb embodied as an open cylinder having a first open end and a second closed end and each barb including at its second closed end a window transparent to optical measuring radiation, wherein each barb is disposed in the port of a respective vessel of the at least two vessels; an optical analyzer having an optical analyzer head, wherein the optical analyzer is connected with the optical analyzer head via a fiber optical cable; a Cartesian robot configured to move parallel to an x-axis and parallel to a mutually orthogonal z-axis, wherein the optical analyzer head is affixed in the Cartesian robot and is enabled to move with the Cartesian robot; and a control unit configured to control the optical analyzer and the Cartesian robot and further configured to coordinate movement of the Cartesian robot and operation of the optical analyzer, wherein the at least two vessels are arranged on the x-axis within a movement range of the Cartesian robot, wherein the control unit is further configured to: move the Cartesian robot and the affixed optical analyzer head along the x-axis from a home position to a position of the port of a first vessel of the at least two vessels; at the position of the port of the first vessel, move the Cartesian robot and the affixed optical analyzer head downward parallel to the z-axis and thereby insert the optical analyzer head into the barb disposed in the port of the first vessel; trigger the optical analyzer to perform an optical analysis of a liquid or gaseous media of the first vessel; move the Cartesian robot and the affixed optical analyzer head upward parallel to the z-axis and thereby extract the optical analyzer head from the barb in the port of the first vessel; move the Cartesian robot and the affixed optical analyzer head along the x-axis to a position of the port of a second vessel of the at least two vessels; repeat the moving downward of the Cartesian robot, the triggering of the optical analyzer, and the moving upward of the Cartesian robot; and return the Cartesian robot to the home position.

According to another embodiment of the present disclosure, the Cartesian robot may be further configured to move parallel to a y-axis that is mutually perpendicular to the x-axis and to the z-axis. The control unit may be further configured to move the Cartesian robot and the affixed optical analyzer head parallel to the x-axis and parallel to the y-axis from the home position to the position of the port of the first vessel, and move the Cartesian robot and the affixed optical analyzer head parallel to the x-axis and parallel to the y-axis to the position of the port of the second vessel.

According to another embodiment of the present disclosure, the system includes at least a third vessel, wherein the third vessel is embodied to contain a liquid or gaseous process, and the third vessel has a port enabling access to an interior of the third vessel, wherein the at least two barbs includes a third barb, and the third barb is disposed in the port of the third vessel, wherein the third vessel is arranged offset from the x-axis in a direction parallel to the y-axis and the first, second, and third vessels thereby form a two-dimensional array of vessels, wherein the control unit is further configured to: move the Cartesian robot parallel to the x-axis and parallel to the y-axis in the two-dimensional array of vessels to a position of the port of the third vessel; at the position of the port of the third vessel, move the Cartesian robot and the affixed optical analyzer head downward parallel to the z-axis and thereby insert the optical analyzer head into the barb disposed in the port of the third vessel; trigger the optical analyzer to perform an optical analysis of a liquid or gaseous media of the third vessel; and move the Cartesian robot and the affixed optical analyzer head upward parallel to the z-axis and thereby extract the optical analyzer head from the barb in the port of the third vessel.

The position of the port of the first vessel and the position of the port of the second vessel may be programmed a priori in the control unit.

According to another embodiment of the present disclosure, the system further comprises a camera mounted on the Cartesian robot and configured to image the first vessel and the second vessel, wherein the position of the port of the first vessel and the position of the port of the second vessel are determined by an artificial intelligence (AI) algorithm using pattern recognition and an image of the first vessel and an image of the second vessel.

The optical analyzer may be a Raman spectroscopic analyzer, the optical analyzer head may be a Raman probe head, and the optical measuring radiation may be laser radiation suitable for Raman spectroscopic analysis.

The at least two vessels may bioreactors.

According to at least one embodiment of the present disclosure, a method for an optical analysis comprises providing a system for the optical analysis, including: at least two vessels, each vessel embodied to contain a liquid or gaseous process and each vessel having a port enabling access to an interior of the respective vessel; at least two barbs, each barb embodied as an open cylinder having a first open end and a second closed end and each barb including at its second closed end a window transparent to optical measuring radiation, wherein each barb is disposed in the port of a respective vessel of the at least two vessels; an optical analyzer having an optical analyzer head, wherein the optical analyzer is connected with the optical analyzer head via a fiber optical cable; a Cartesian robot configured to move parallel to an x-axis and parallel to a mutually orthogonal z-axis, wherein the optical analyzer head is affixed in the Cartesian robot and is enabled to move with the Cartesian robot; and a control unit configured to control the optical analyzer and the Cartesian robot and further configured to coordinate movement of the Cartesian robot and operation of the optical analyzer, wherein the at least two vessels are arranged on the x-axis within a movement range of the Cartesian robot, wherein the control unit is further configured to: move the Cartesian robot and the affixed optical analyzer head along the x-axis from a home position to a position of the port of a first vessel of the at least two vessels; at the position of the port of the first vessel, move the Cartesian robot and the affixed optical analyzer head downward parallel to the z-axis and thereby insert the optical analyzer head into the barb disposed in the port of the first vessel; trigger the optical analyzer to perform an optical analysis of a liquid or gaseous media of the first vessel; move the Cartesian robot and the affixed optical analyzer head upward parallel to the z-axis and thereby extract the optical analyzer head from the barb in the port of the first vessel; move the Cartesian robot and the affixed optical analyzer head along the x-axis to a position of the port of a second vessel of the at least two vessels; repeat the moving downward of the Cartesian robot, the triggering of the optical analyzer, and the moving upward of the Cartesian robot; and return the Cartesian robot to the home position; moving the Cartesian robot and the affixed optical analyzer head along the x-axis from the home position to the position of the port of the first vessel; at the position of the port of the first vessel, moving the Cartesian robot and the affixed optical analyzer head downward parallel to the z-axis and thereby insert the optical analyzer head into the barb disposed in the port of the first vessel; triggering the optical analyzer to perform the optical analysis of a liquid or gaseous media of the first vessel; moving the Cartesian robot and the affixed optical analyzer head upward parallel to the z-axis and thereby extract the optical analyzer head from the barb in the port of the first vessel; moving the Cartesian robot and the affixed optical analyzer head along the x-axis to the position of the port of the second vessel; repeating the moving downward of the Cartesian robot, the triggering of the optical analyzer, and the moving upward of the Cartesian robot; and returning the Cartesian robot to the home position.

According to an embodiment of the method of the present disclosure, the Cartesian robot may be further configured to move parallel to a y-axis that is mutually perpendicular to the x-axis and to the z-axis, the control unit may be further configured to move the Cartesian robot and the affixed optical analyzer head parallel to the x-axis and parallel to the y-axis from the home position to the position of the port of the first vessel, and move the Cartesian robot and the affixed optical analyzer head parallel to the x-axis and parallel to the y-axis to the position of the port of the second vessel, wherein the step of moving the Cartesian robot from the home position to the position of the port of the first vessel may include moving the Cartesian robot and the affixed optical analyzer head parallel to the x-axis and parallel to the y-axis from the home position to the position of the port of the first vessel, and wherein the step of moving the Cartesian robot and the affixed optical analyzer to the position of the port of the second vessel may include moving the Cartesian robot and the affixed optical analyzer head parallel to the x-axis and parallel to the y-axis to the position of the port of the second vessel.

The system may further include a third vessel embodied to contain a liquid or gaseous process, and the third vessel may have a port enabling access to an interior of the third vessel, the system may further include a third barb, and the third barb may be disposed in the port of the third vessel, the third vessel may be arranged offset from the x-axis in a direction parallel to a y-axis that is mutually perpendicular to the x-axis and to the z-axis, the Cartesian robot may be further configured to move parallel to the y-axis, and the method may further comprise moving the Cartesian robot parallel to the x-axis and parallel to the y-axis to a position of the port of the third vessel; at the position of the port of the third vessel, moving the Cartesian robot and the affixed optical analyzer head downward parallel to the z-axis and thereby insert the optical analyzer head into the barb disposed in the port of the third vessel; triggering the optical analyzer to perform an optical analysis of a liquid or gaseous media of the third vessel; and moving the Cartesian robot and the affixed optical analyzer head upward parallel to the z-axis and thereby extract the optical analyzer head from the barb in the port of the third vessel.
Fig. 1 shows a system according to an embodiment of the present disclosure.
Fig. 2 shows a Raman probe head placed in a disposable barb according to an embodiment of the present disclosure.
Fig. 3 shows the Raman probe after placement into the barb according to an embodiment of the present disclosure.
Fig. 4 shows a Raman probe placed in a barb according to the state of the art.
Fig. 5 shows a method of analyzing the bioreactors according to an embodiment of the present disclosure.

Disclosed herein is a system and method for performing Raman analyses on the contents of individual bioreactors in an array of bioreactors using only a single Raman probe. Various embodiments of the disclosed system and method will now be presented in conjunction with the figures that illustrate the embodiments. It will be understood that no limitation of the scope of this disclosure is thereby intended.

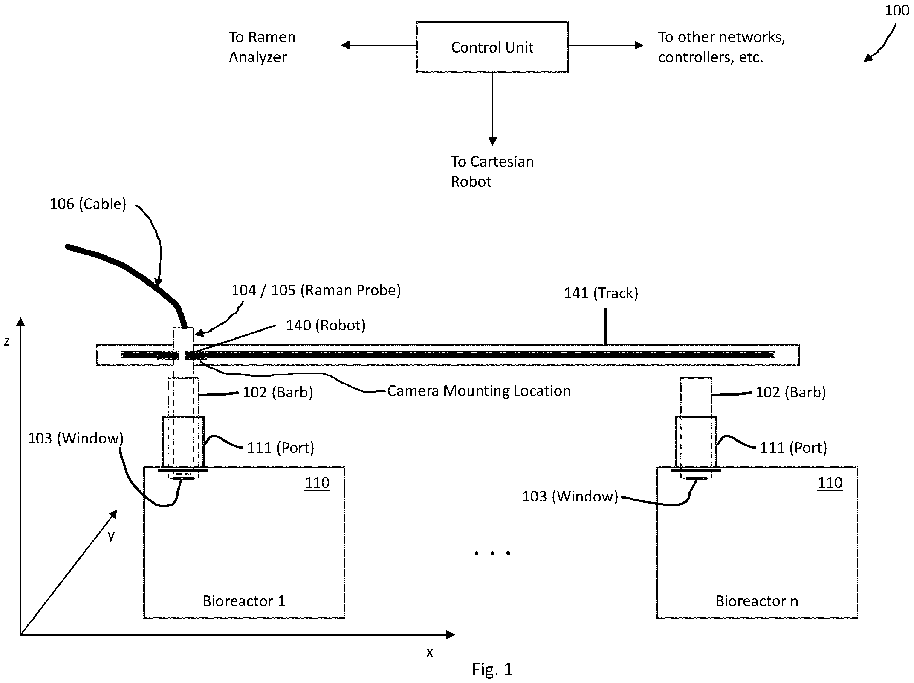

Fig. 1 shows schematically a simplified system 100 according to an embodiment of the present disclosure. In the system 100 may be an array of 'n' bioreactors 110, wherein 'n' is greater than 1. Integrated into each bioreactor 110 in the array may be a port 111 that may enable access to the inside of the bioreactor 110, and such access may permit the addition of contents to the bioreactor 110 or the extraction of samples from the bioreactor 110 or the insertion of some measuring instrument into the bioreactor 110.

Inserted into each port 111 of each bioreactor 110 in the system 100 may be a barb 102. The barb 102 may have an open cylindrical design with an open first end and a closed second end. The closed second end of the barb 102 may include a window 103 that is transparent to light used in Raman measurements. The barb 102 may be inserted into the port 111 so that the barb 102 extends entirely through the port and the window 103 may thus be disposed within the bioreactor 110.

Such a barb 102 is disclosed in U.S. Pat. No 10,261,020 issued to Kaiser Optical Systems, Inc., of Ann Arbor, MI, USA. The entire contents of U.S. Pat. No 10,261,020 is incorporated herein by reference.

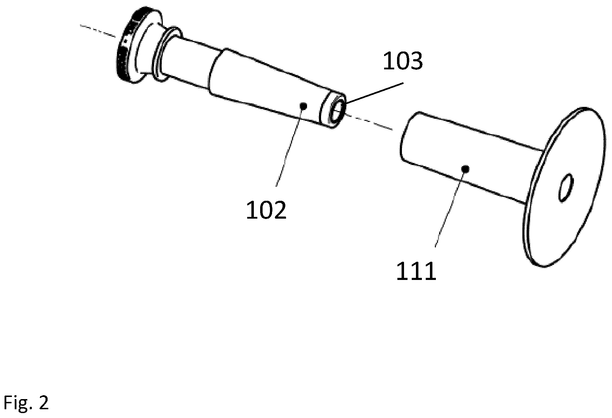

The barb 102 with its window 103 is shown in Fig. 2. Also shown in Fig. 2 is port 111 which is shown separately from the bioreactor 110 for clarity.

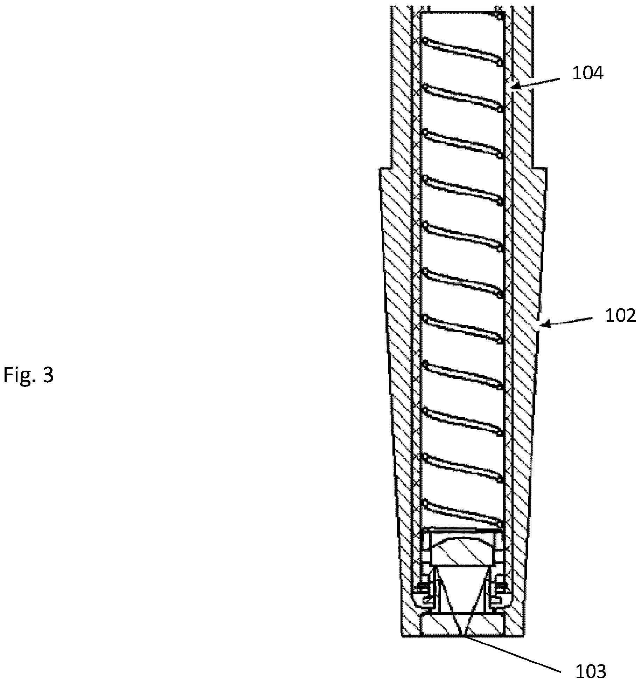

The barb 102 may be embodied to hold a Raman probe 105 optic component 104. That is, the interior of the barb 102 -- including its first open end -- may be dimensioned to snugly hold the optic component 104 so that the end of the optic component 104 is disposed adjacent to the window 103. A barb 102 with the optic component 104 inserted is shown in Fig. 3.

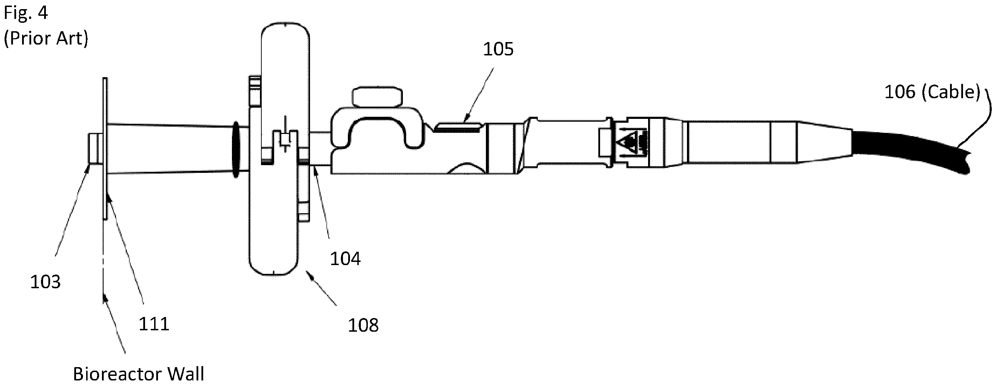

Fig. 4 shows the relation between the Raman probe 105, the optic component 104, the barb 102, the window 103 of the barb 102, and the bioreactor port 111. In this manual placement of the optic component 104 as known in the art, the barb 102 is not necessarily seen within the port 111, but the window 103 of the barb 102 may extend through the port 111 and into the interior of the bioreactor 110, if only a little bit. Taking Figs. 3 and 4 together then, the placement of the barb 102 and the optic component 104 may place the tip of the optic component 104 of the Raman probe 105 close to the medium within the bioreactor 110.

Also shown in Fig. 4 is a retainer 108 that may be used to hold the Raman probe 105 in place at the bioreactor port 111. However, in the context of the present disclosure, such a retainer 108 is not a part of the system 100.

Though the optic component 104 may fit snugly within the barb 102 (as shown in Fig. 3), the Raman probe 105 is not permanently fixed within the barb 102, but rather it may be freely removed from the barb 102 (and freely inserted into the barb 102). Indeed, that the Raman probe 105 may be freely inserted into the barb 102 and freely removed from the barb 102 enables the method of the present disclosure: the same Raman probe 105 and the same optic component 104 may be used with the 'n' barbs 102 of the system 100.

The system 100 may further include a Cartesian robot 140 moveable in at least two mutually orthogonal directions, and optionally moveable in three mutually orthogonal directions. In the system 100 as shown in Fig. 1, the Cartesian robot 140 is moveable along the track 141 that is aligned on the x-axis, and the Cartesian robot 140 may also be moveable along the z-axis. Though Cartesian robots as known in the art may be moveable along the x, y, and z axes, movements along only the x and z axes are shown in the system 100 of Fig. 1 for clarity.

The Raman probe 105 and the optic component 104 may be affixed in the Cartesian robot 140, and therefore movement of the Cartesian robot along the z and x axes moves the Raman probe 105 and the optic component 104 as well. For example, moving the Cartesian robot along the x-axis may also move the Raman probe 105 and the optic component 104 along the x-axis, and such movement may be used to move the Raman probe 105 and the optic component 104 to and from each of the bioreactors 110 in the array of bioreactors. Additionally, movement of the Cartesian robot along the z-axis may be used to move the optic component 104 into and out of a barb 102 disposed within a port 111 of a bioreactor 110.

That is to say, in a system 100 having an array of bioreactors, the Cartesian robot 140 may be used to move the Raman probe 105 and the optic component 104 to a particular bioreactor 110 in the array of bioreactors, and, once there, move the optic component 104 into the barb 102 and thereby enable a Raman analyzer to perform a Raman analysis of the contents of that bioreactor 110 via the Raman probe 105 and the optic component 104. Once the Raman analysis of the particular bioreactor 110 is complete, the Cartesian robot may be used to move the optic component 104 out of the barb 102 and then to another bioreactor 110 in the array of bioreactors, and once at the other bioreactor, to place the optic component 104 into the barb 102 of the other bioreactor for a subsequent Raman analysis.

In the system 100 as shown in Fig. 1, the array of bioreactors is an 'n' x 1 array, but the one-dimensional array is used only for ease of illustration. As easily as an 'n' x 1 array, an 'n' x 'm' two-dimensional array of bioreactors may be used in the system 100. Of course the Cartesian robot 140 would need to be configured to move additionally in the y direction, and the fiber optic cable 106 connecting the optic component 104 to the Raman analyzer would need to be sufficiently long to reach each of the bioreactors 110 in the 'n' x 'm' array of bioreactors. But the basic method enabled by the system remains the same: move, place, analyze, remove, and repeat.

Although the system as shown in Fig. 1 is an 'n' x 1 array (i.e., one dimensional), it may require movement in the x and y directions to place the optical component 104 into a barb 102. The reasons for the two-dimensional movement along a one-dimensional array may be varied. It may be difficult to align all the ports of all the bioreactors along a single line; or it may be necessary to move around obstacles in the path of movement of the Cartesian robot 140 (and the optical component 104).

Movement of the Cartesian robot 140 from bioreactor 110 to bioreactor 110 may be most easily done when the positions of the bioreactors are programmed into the controller of the Cartesian robot. The location of each bioreactor 110 and the position and depth of each barb 102 may be so programmed. Note there is no requirement for a particular location for a bioreactor 110 except within reach of the Cartesian robot 140, but only that the individual locations or the bioreactors 110 be programmed into the robot's controller.

However, the programming of these geographic locations is not strictly necessary. Instead, the training of an artificial intelligence (AI) algorithm to recognize from images an individual bioreactor 110 and its port 111 (and the barb 102 within the port 111) may be used. In a system 100 that uses such AI methods to place the Raman probe 105 and the optic component 104, a camera mounted on the Cartesian robot 140 is helpful. For example, a bioreactor 110 may include a Quick Response (QR) code or other scannable code on the bioreactor exterior that may identify the type of the bioreactor 110, the process parameters, etc., and the placement and content of this scannable code may be used by an Al algorithm in the placement of the Cartesian robot 140 and thus the Raman probe 105 in the bioreactor 110.

The system 100 may also include a central control unit that is embodied to coordinate together the Raman analysis performed on the contents of the various bioreactors 110 and the movement of the Raman probe head 104 from bioreactor 110 to bioreactor 110.

Therefore the central control unit may include a processor and sufficient memory to execute at least a control algorithm. The central control unit may include one or more communication interfaces to enable communication with the Raman analyzer, the Cartesian robot, any Al system, a higher-level controller, etc.

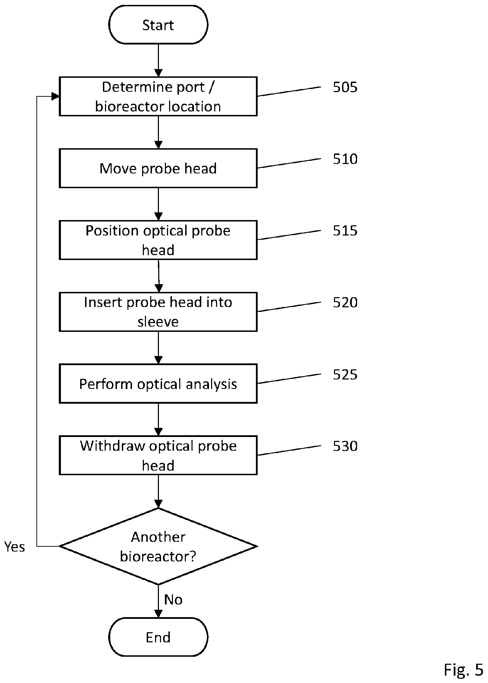

Fig. 5 shows a method 500 of performing a Raman analysis on the contents of each bioreactor in an array of bioreactors (in a system as shown in Fig. 1, for example). The method 500 may include a step 505 of determining the position of a bioreactor / port / barb for the placement of a Raman probe into the barb. The positions of the bioreactors / ports / barbs may be determined and programmed *a priori,* or they may be determined during the running of the method 500 by the use of Al and image recognition.

For example, an Al algorithm may be trained on the appearance of the bioreactors and the appearance of the port of a bioreactor with a barb inserted therein. Alternately or additionally, the Al algorithm may be trained to recognize and scan an optical code on a bioreactor identifying that bioreactor and therefrom determine the placement of the port of the bioreactor with the barb placed therein.

But alternately, the positions of the bioreactors in the array of bioreactors and the positions of each port on each bioreactor may be programmed into the control algorithm of the Cartesian robot.

Note that either method -- pre-programming bioreactor locations or using pattern recognition -- may be used with bioreactors of different sizes and shapes in the same array of bioreactors. The method does not require uniformity in bioreactor type, size, or even placement.

The method 500 may then include a step 510 of moving a Cartesian robot holding a Raman probe to the location so determined for the bioreactor / port / barb. Once there, the method 500 may include a step 515 of positioning the Raman probe with the optic component over the open barb in the port of the bioreactor.

Once the Raman probe has been so positioned, the method 500 may include a step 520 of inserting the optic component into the barb that is in the port of the bioreactor. The method step 520 places the optic component within the barb so that the end of the optic component is well within the barb, adjacent to the window disposed at the end of the barb. Thus the Raman probe is placed as close to the contents of the bioreactor as the barb will allow.

With the optic component placed within the barb, the method 500 may include a step 525 of performing a Raman analysis of the contents within the bioreactor. The Raman analysis is as known in the art: a [likely] monochromatic light is shone into the media of the bioreactor via the Raman probe; light scattered by the media is collected by the Raman probe; and an analysis of the collected light is made to determine what substances within media within the bioreactor scattered that light.

Once the illumination of the sample and the gathering of the scattered light is complete in step 525, the method 500 may include a step 530 of withdrawing the optic component from the barb. Note the Raman analysis of the scattered light need not be completed before the optic component is withdrawn from the barb.

The method 500 may at this point finish if no further bioreactors in the array of bioreactors need analyzed. However, if there are additional bioreactors to analyze, the method may return to step 505 of determining the position of the next bioreactor / port / barb for subsequent Raman analysis. The position of the next bioreactor / port / barb may be in a list of positions as determined *a priori,* or the next position may be determined by the Al algorithm using images captured from a camera mounted on the Cartesian robot.

Though Raman analysis of the contents of the various bioreactors is given as the example embodiments of both the system and the method, the system and method of the present disclosure are not limited to Raman analysis or even bioreactors. For example, other methods of optical analysis such as infrared absorption spectroscopy may be used. In this case, of course, the window 103 in the barb 102 must necessarily be transparent to the radiation used in the particular optical analysis.

Additionally, other types of vessels may be used in place of the bioreactors. For example, the method according to the present disclosure may be used in an array of electrolytic cells in which Raman or other optical analysis of the contents of the various cells must be performed.

Additionally, although the vessels in the exemplary embodiments of both the system and the method have been arranged in an array (e.g., a 1 x 'n' array or an 'n' x 'm' array) and the robot of these exemplary embodiments is a Cartesian robot, such is not a necessity for the disclosed system or method. For example, it may be that a circular or similar arrangement of the vessels is more convenient or more suitable for a given system or application, and that a robot having *polar* movement is best suited for the arrangement of vessels. For example, a SCARA robot (i.e., Selective Compliance Assembly Robot Arm) with its rotating motion may be especially suitable for a circular arrangement of vessels into which placement of the optical component requires a generally vertical movement. But indeed other suitable arrangements of the vessels and other suitable robots having various rotational or prismatic joints may be used within the scope of the present disclosure.

## Claims

1. A system for optical analysis, comprising:
at least two vessels, each vessel embodied to contain a liquid or gaseous process and each vessel having a port enabling access to an interior of the respective vessel;
at least two barbs, each barb embodied as an open cylinder having a first open end and a second closed end and each barb including at its second closed end a window transparent to optical measuring radiation, wherein each barb is disposed in the port of a respective vessel of the at least two vessels;
an optical analyzer having an optical analyzer head, wherein the optical analyzer is connected with the optical analyzer head via a fiber optical cable;
a Cartesian robot configured to move parallel to an x-axis and parallel to a mutually orthogonal z-axis, wherein the optical analyzer head is affixed in the Cartesian robot and is enabled to move with the Cartesian robot; and
a control unit configured to control the optical analyzer and the Cartesian robot and further configured to coordinate movement of the Cartesian robot and operation of the optical analyzer,
wherein the at least two vessels are arranged on the x-axis within a movement range of the Cartesian robot,
wherein the control unit is further configured to:
move the Cartesian robot and the affixed optical analyzer head along the x-axis from a home position to a position of the port of a first vessel of the at least two vessels;
at the position of the port of the first vessel, move the Cartesian robot and the affixed optical analyzer head downward parallel to the z-axis and thereby insert the optical analyzer head into the barb disposed in the port of the first vessel;
trigger the optical analyzer to perform an optical analysis of a liquid or gaseous media of the first vessel;
move the Cartesian robot and the affixed optical analyzer head upward parallel to the z-axis and thereby extract the optical analyzer head from the barb in the port of the first vessel;
move the Cartesian robot and the affixed optical analyzer head along the x-axis to a position of the port of a second vessel of the at least two vessels;
repeat the moving downward of the Cartesian robot, the triggering of the optical analyzer, and the moving upward of the Cartesian robot; and
return the Cartesian robot to the home position.

2. The system of claim 1,
wherein the Cartesian robot is further configured to move parallel to a y-axis that is mutually perpendicular to the x-axis and to the z-axis, and
wherein the control unit is further configured to:
move the Cartesian robot and the affixed optical analyzer head parallel to the x-axis and parallel to the y-axis from the home position to the position of the port of the first vessel, and
move the Cartesian robot and the affixed optical analyzer head parallel to the x-axis and parallel to the y-axis to the position of the port of the second vessel.

3. The system of claim 2,
wherein the at least two vessels includes at least a third vessel,
wherein the third vessel is embodied to contain a liquid or gaseous process, and the third vessel has a port enabling access to an interior of the third vessel,
wherein the at least two barbs includes a third barb, and the third barb is disposed in the port of the third vessel,
wherein the third vessel is arranged offset from the x-axis in a direction parallel to the y-axis and the first, second, and third vessels thereby form a two-dimensional array of vessels,
wherein the control unit is further configured to:
move the Cartesian robot parallel to the x-axis and parallel to the y-axis in the two-dimensional array of vessels to a position of the port of the third vessel;
at the position of the port of the third vessel, move the Cartesian robot and the affixed optical analyzer head downward parallel to the z-axis and thereby insert the optical analyzer head into the barb disposed in the port of the third vessel;
trigger the optical analyzer to perform an optical analysis of a liquid or gaseous media of the third vessel; and
move the Cartesian robot and the affixed optical analyzer head upward parallel to the z-axis and thereby extract the optical analyzer head from the barb in the port of the third vessel.

4. The system of claim 1,
wherein the position of the port of the first vessel and the position of the port of the second vessel are programmed a priori in the control unit.

5. The system of claim 2, further comprising:
a camera mounted on the Cartesian robot and configured to image the first vessel and the second vessel,
wherein the position of the port of the first vessel and the position of the port of the second vessel are determined by an artificial intelligence (AI) algorithm using pattern recognition and an image of the first vessel and an image of the second vessel.

6. The system of claim 1,
wherein the optical analyzer is a Raman spectroscopic analyzer, and the optical analyzer head is a Raman probe head, and
wherein the optical measuring radiation is laser radiation suitable for Raman spectroscopic analysis.

7. The system of claim 1,
wherein the at least two vessels are bioreactors.

8. A method for an optical analysis, comprising:
providing a system for the optical analysis, including:
at least two vessels, each vessel embodied to contain a liquid or gaseous process and each vessel having a port enabling access to an interior of the respective vessel;
at least two barbs, each barb embodied as an open cylinder having a first open end and a second closed end and each barb including at its second closed end a window transparent to optical measuring radiation, wherein each barb is disposed in the port of a respective vessel of the at least two vessels;
an optical analyzer having an optical analyzer head, wherein the optical analyzer is connected with the optical analyzer head via a fiber optical cable;
a Cartesian robot configured to move parallel to an x-axis and parallel to a mutually orthogonal z-axis, wherein the optical analyzer head is affixed in the Cartesian robot and is enabled to move with the Cartesian robot; and
a control unit configured to control the optical analyzer and the Cartesian robot and further configured to coordinate movement of the Cartesian robot and operation of the optical analyzer,
wherein the at least two vessels are arranged on the x-axis within a movement range of the Cartesian robot,
wherein the control unit is further configured to:
move the Cartesian robot and the affixed optical analyzer head along the x-axis from a home position to a position of the port of a first vessel of the at least two vessels;
at the position of the port of the first vessel, move the Cartesian robot and the affixed optical analyzer head downward parallel to the z-axis and thereby insert the optical analyzer head into the barb disposed in the port of the first vessel;
trigger the optical analyzer to perform an optical analysis of a liquid or gaseous media of the first vessel;
move the Cartesian robot and the affixed optical analyzer head upward parallel to the z-axis and thereby extract the optical analyzer head from the barb in the port of the first vessel;
move the Cartesian robot and the affixed optical analyzer head along the x-axis to a position of the port of a second vessel of the at least two vessels;
repeat the moving downward of the Cartesian robot, the triggering of the optical analyzer, and the moving upward of the Cartesian robot; and
return the Cartesian robot to the home position;
moving the Cartesian robot and the affixed optical analyzer head along the x-axis from the home position to the position of the port of the first vessel;
at the position of the port of the first vessel, moving the Cartesian robot and the affixed optical analyzer head downward parallel to the z-axis and thereby insert the optical analyzer head into the barb disposed in the port of the first vessel;
triggering the optical analyzer to perform the optical analysis of a liquid or gaseous media of the first vessel;
moving the Cartesian robot and the affixed optical analyzer head upward parallel to the z-axis and thereby extract the optical analyzer head from the barb in the port of the first vessel;
moving the Cartesian robot and the affixed optical analyzer head along the x-axis to the position of the port of the second vessel;
repeating the moving downward of the Cartesian robot, the triggering of the optical analyzer, and the moving upward of the Cartesian robot; and
returning the Cartesian robot to the home position.

9. The method of claim 8,
wherein the Cartesian robot is further configured to move parallel to a y-axis that is mutually perpendicular to the x-axis and to the z-axis,
wherein the control unit is further configured to:
move the Cartesian robot and the affixed optical analyzer head parallel to the x-axis and parallel to the y-axis from the home position to the position of the port of the first vessel, and
move the Cartesian robot and the affixed optical analyzer head parallel to the x-axis and parallel to the y-axis to the position of the port of the second vessel,
wherein the step of moving the Cartesian robot from the home position to the position of the port of the first vessel includes moving the Cartesian robot and the affixed optical analyzer head parallel to the x-axis and parallel to the y-axis from the home position to the position of the port of the first vessel, and
wherein the step of moving the Cartesian robot and the affixed optical analyzer to the position of the port of the second vessel includes moving the Cartesian robot and the affixed optical analyzer head parallel to the x-axis and parallel to the y-axis to the position of the port of the second vessel.

10. The method of claim 9,
wherein the at least two vessels includes at least a third vessel,
wherein the third vessel is embodied to contain a liquid or gaseous process, and the third vessel has a port enabling access to an interior of the third vessel,
wherein the at least two barbs includes a third barb, and the third barb is disposed in the port of the third vessel,
wherein the third vessel is arranged offset from the x-axis in a direction parallel to the y-axis and the first, second, and third vessels thereby form a two-dimensional array of vessels,
the method further comprising:
moving the Cartesian robot parallel to the x-axis and parallel to the y-axis in the two-dimensional array of vessels to a position of the port of the third vessel;
at the position of the port of the third vessel, moving the Cartesian robot and the affixed optical analyzer head downward parallel to the z-axis and thereby insert the optical analyzer head into the barb disposed in the port of the third vessel;
triggering the optical analyzer to perform an optical analysis of a liquid or gaseous media of the third vessel; and
moving the Cartesian robot and the affixed optical analyzer head upward parallel to the z-axis and thereby extract the optical analyzer head from the barb in the port of the third vessel.
